# EUROPÄISCHE PATENTSCHRIFT

(11) **EP 3 046 492 B1**
(45) Veröffentlichungstag und Bekanntmachung des Hinweises auf die Patenterteilung: **08.08.2018**
(21) Anmeldenummer: 14771834.0
(22) Anmeldetag: 17.09.2014
(51) Int. Cl.: A61B 18/04, A61B 18/14, C23C 26/00, C23C 16/00

(54) **SPANNUNGSFESTE, ELEKTRISCH ISOLIERENDE BESCHICHTUNGEN**
VOLTAGE-RESISTANT, ELECTRICALLY INSULATING COATINGS
REVÊTEMENTS D'ISOLATION ÉLECTRIQUE RÉSISTANT AUX TENSIONS

(30) Priorität: 20.09.2013 DE 102013110394
(43) Veröffentlichungstag der Anmeldung: 27.07.2016
(73) Patentinhaber: NMI Naturwissenschaftliches und Medizinisches Institut an der Universität Tübingen, 72770 Reutlingen (DE)
(72) Erfinder: BUCHER, Volker, 78628 Rottweil (DE); NISCH, Wilfried, 72070 Tuebingen (DE); ROEDER, Marcel, 73630 Remshalden (DE)
(74) Vertreter: Witte, Weller & Partner Patentanwälte mbB
(86) Internationale Anmeldenummer: PCT/EP2014/069780
(87) Internationale Veröffentlichungsnummer: WO 2015/040048

(56) Entgegenhaltungen:
- EP-A1- 0 517 244
- US-A- 5 702 387
- US-A- 5 925 039
- US-A1- 2001 012 936
- US-A1- 2005 154 385
- US-A1- 2006 025 757
- US-A1- 2007 005 060
- US-A1- 2011 122 486
- US-B2- 6 926 572
- US-B2- 6 999 818

## Beschreibung

Die vorliegende Erfindung betrifft ein chirurgisches Instrument, insbesondere ein elektrochirurgisches Instrument, mit einer Oberfläche, auf der eine elektrisch isolierende Beschichtung vorgesehen ist, sowie ein Verfahren zur Herstellung derartiger Beschichtungen.

US 2001/0012936 A1 zeigt ein elektrochirurgisches Gerät, das aus einem metallischen Grundkörper besteht, der mit einer Isolationsschicht überzogen ist, die den Grundkörper thermisch und elektrisch isolieren soll.

US 2005/0154385 A1 zeigt ebenfalls ein elektrochirurgisches Instrument mit einer Isolationsschicht.

US 2007/005060 offenbart ein weiteres elektrochirurgisches Instrument mit einer Isolationsschicht, die thermische und elektrische Entladungen reduzieren und das Instrument isolieren soll.

Spannungsfeste, elektrisch isolierende Oberflächenbeschichtungen werden in vielen Bereichen der Technik benötigt. Sie schützen die Oberflächen, auf denen die Beschichtung aufgebracht ist, nicht nur vor dem unmittelbaren Kontakt mit elektrischen Spannungen, sie verhindern auch, dass über den Beschichtungen anliegende elektrische Spannungen auf die Oberflächen "durchschlagen".

Wenn derartige Isolationsschichten versagen, spricht man allgemein von einem Spannungsdurchschlag.

Damit diese Beschichtungen ihre Schutzfunktion dauerhaft ausüben können, sollen sie mechanisch belastbar, insbesondere kratzfest, abriebfest und verschleißfest sein, so dass sie bei den erforderlichen Reinigungsmaßnahmen und/oder im Gebrauch nicht allmählich abgetragen werden oder Beschädigungen erleiden, die die Spannungsfestigkeit beeinträchtigen.

Schon kleinste Defekte in der Beschichtung, beispielsweise Risse, Löcher oder Vertiefungen, führen nämlich dazu, dass lokal im Bereich dieser Oberflächenbeschädigungen die Spannungsfestigkeit nicht mehr gegeben ist.

Anwendung finden derartige Beschichtungen in und auf elektrisch betriebenen Geräten und/oder damit in Kontakt gelangenden Gegenständen. Sie dienen dort dem Schutz der Geräte, der Gegenstände und der damit in Kontakt gelangenden Personen oder Tiere vor Kontakt mit elektrischen Spannungen und/oder Strömen.

Unter einer "Vorrichtung" wird im Rahmen der vorliegenden Erfindung daher ein Gegenstand beliebiger Art verstanden, der eine äußere oder innere Oberfläche aufweist, die mit einer elektrisch isolierenden Beschichtung versehen wird oder ist.

Nachstehend werden "spannungsfeste, elektrisch isolierende Beschichtungen" auch verkürzt als "Isolationsschichten" bezeichnet.

Besonders wichtig sind derartige Beschichtungen in der Elektrochirurgie, wo sie dem Schutz des Patienten und des Operateurs dienen. Die Elektrochirurgie ist ein wichtiges Operationsverfahren, bei dem unter Verwendung von hochfrequentem Wechselstrom beispielsweise Blutungen gestillt werden und Gewebe geschnitten oder versiegelt wird.

Elektrochirurgische Instrumente werden nach der DIN Norm 60601-2-2 geprüft. Die verwendeten Isolationsschichten müssen danach Spannungen von über 1000 Volt standhalten.

Als elektrische Isolationsschichten werden aktuell meist Kunststoffe und Keramiken verwendet. Eine häufig anzutreffende Beschichtung besteht aus Polyamid 11 oder 12 (Rilsan®), das durch eine Pulverbeschichtung auf die Instrumente aufgebracht wird. Polyamid ist ein thermoplastischer Kunststoff und wird durch die Polykondensation aus Dicarbonsäure und Diaminen hergestellt.

Weitere oft verwendete Materialien sind die Hochleistungskunststoffe Polyetheretherketon (PEEK) und Polytetrafluorethylen (PTFE, Teflon®). Ein anderes Verfahren zur Isolierung elektrochirurgischer Instrumente ist die Verwendung von Schrumpfschläuchen aus Nylon oder Polyimid.

Der Vorteil von Kunststoffen als Isolationsmaterial ist eine gute Resistenz gegen Schlag- und Stoßbeanspruchung. Probleme entstehen jedoch bei hoher thermischer Belastung der Kunststoffe.

Neben der Gewährleistung der erforderlichen Spannungsfestigkeit von vorzugsweise mindestens 1.000 Volt müssen die Beschichtungen biokompatibel, wiederholt sterilisierbar und mechanisch beanspruchbar sein.

Die bekannten Isolationsschichten, die diese Forderungen erfüllen, weisen in der Regel alle eine minimale Stärke von 0,15 mm auf, häufig werden Isolationsschichten mit einer Mindeststärke von 0,25 mm eingesetzt.

Die Chirurgie ist heute bestrebt, Operationen mit immer kleineren Eingriffen durchführen zu können, um Traumata für den Patienten minimal zu halten. Neue minimalinvasive Operationseingriffe im Bereich der Neurochirurgie, der Urologie, der interventionellen Kardiologie und der Gynäkologie benötigen zudem miniaturisierte Instrumente, um Operationen an schwer erreichbaren Gebieten vornehmen zu können.

Die bekannten Isolationsschichten tragen zu dem Durchmesser eines elektrochirurgischen Instrumentes jedoch in der Regel bereits zwei Mal 0,25 mm, also ca. 0,5 mm bei, stehen also einer weitergehenden Miniaturisierung der Instrumente entgegen.

Daher werden dünnere Isolationsschichten mit vergleichbaren Eigenschaften benötigt, wie sie die bekannten Beschichtungen aufweisen.

Die Erfindung ist in den Ansprüchen 1 und 13 definiert. Weitere Aspekte und bevorzugte Ausführungen sind in den beigefügten Ansprüchen definiert. Aspekte, Ausführungen und Beispiele der vorliegenden Offenbarung, die nicht in den Anwendungsbereich der beigefügten Ansprüche fallen, sind nicht Bestandteil der Erfindung und dienen lediglich der Veranschaulichung.

Der vorliegenden Erfindung liegt daher die Aufgabe zugrunde, eine neue Beschichtung der eingangs genannten Art zu schaffen, die eine Stärke von weniger als 0,1 mm aufweist, aber vergleichbare elektrische und mechanische Eigenschaften wie die bekannten Beschichtungen zeigt.

Diese Aufgabe wird erfindungsgemäß dadurch gelöst, dass die Beschichtung mehrlagig ausgebildet ist, mit zumindest einer auf einer Oberfläche des chirurgischen Instruments aufgebrachten unteren Schicht, die eine Stärke von höchstens 50 um aufweist, und einer auf der unteren Schicht aufgebrachten, oberen Schicht, die eine Stärke von höchstens 50 um aufweist, wobei die untere Schicht aus einem spannungsfesten harten Material und die obere Schicht aus einem spaltgängigen spannungsfesten Material besteht, wobei die Beschichtung eine Spannungsfestigkeit zumindest bis 1.000 Volt aufweist.

Unter einem" harten spannungsfesten Material" wird im Rahmen der vorliegenden Erfindung ein Material verstanden, das bei einer Stärke unterhalb von 50 um eine theoretische Spannungsfestigkeit von mindestens 1.000 Volt aufweist, und das eine Kratz- und Verschleißfestigkeit aufweist wie DLC (diamond-like carbon), das beim Abscheiden auf der Oberfläche aber infolge von Kondensationseffekten, Partikeleinwirkung oder Schichtspannungsrissen Defekte in Form von Sacklöchern und/oder bis zur Oberfläche durchgehenden Löchern ausbildet, die die Spannungsfestigkeit beeinträchtigen.

Beispiele für im Rahmen der vorliegenden Erfindung verwendbare harte spannungsfeste Materialien sind neben Al₂O₃, DLC und SiOₓ auch SiNₓ, Ta₂O₅, HfO₂, TiO₂, ZrO₂.

Unter einem "spaltgängigen spannungsfesten Material" wird im Rahmen der vorliegenden Erfindung ein Material verstanden, das beim Abscheiden auf der Schicht aus hartem Material die in dem harten Material vorhandenen Defekte auffüllt, vorzugsweise sterilisierbar ist, und bei einer Stärke, die der des harten spannungsfesten Materials entspricht, eine theoretische Spannungsfestigkeit von mindestens 1.000 Volt aufweist, aber wegen der Spaltgängigkeit mechanisch weicher als DLC oder SiOₓ ist.

Beispiele für im Rahmen der vorliegenden Erfindung verwendbare spaltgängige spannungsfeste Materialien sind allgemein weiche Polymere wie Parylene oder mit dem speziellen Atomic Layer Deposition Verfahren (ALD) aufgebrachte harte Materialien.

Unter einer "theoretischen Spannungsfestigkeit" wird im Rahmen der vorliegenden Erfindung die Spannungsfestigkeit verstanden, die unter idealen Bedingungen bei einer mechanisch intakten Schicht der entsprechenden Dicke oder Stärke erreicht wird.

Die Erfinder haben also zwei für sich genommen nicht für eine Beschichtung von elektrochirurgischen Instrumenten geeignete Beschichtungsmaterialien zu einer mehrlagigen Beschichtung kombiniert, in der die mechanischen Defekte in der unteren Schicht durch die spaltgängige obere Schicht aufgefüllt werden. Die Spannungsfestigkeit wird dabei vorrangig durch Material und Dicke der unteren Schicht bestimmt.

Die erfindungsgemäße mehrlagige Beschichtung ist wegen der Eigenschaften der oberen Schicht sterilisierbar. Die Tatsache, dass die obere Schicht ggf. nicht so abriebfest ist wie die untere, kann dazu führen, dass die obere Schicht im Laufe der Zeit ganz oder teilweise abgetragen wird. Der Verschluss der Defekte in der unteren Schicht bleibt dennoch erhalten, so dass wegen des ebenfalls spannungsfesten Füllmaterials die Spannungsfestigkeit der neuen Beschichtung dennoch erhalten bleibt.

Weil das Material der oberen Schicht in den Defekten von dem härteren Material der unteren Schicht umgeben ist, wird dieses Füllmaterial selbst nach vollständigem Entfernen der oberen Schicht bei weiteren Beanspruchungen durch Sterilisieren und mechanische Belastungen nicht weiter entfernt.

Wenn die Stärke der unteren Schicht so groß ist, dass eine Schicht aus dem oberen Material gleicher Stärke die erforderliche Spannungsfestigkeit aufweist, bleibt auch nach Abtragung der oberen Schicht die Spannungsfestigkeit erhalten, weil die Defekte in der unteren Schicht mit dem Material der oberen Schicht verfüllt sind.

Die erfindungsgemäße Beschichtung weist eine Stärke von 100 um oder weniger auf, so dass sie weiter miniaturisierte elektrochirurgische Instrumente ermöglicht. In einem bevorzugten Ausführungsbeispiel weisen die untere und die obere Schicht jeweils eine Stärke von ca. 5 um auf. In weiteren bevorzugten Ausführungsbeispielen weist die unter Schicht eine Stärke von ca. 10 um bis 16 um und die obere Schicht eine Stärke von ca. 8 um bis 10 um auf.

Vor diesem Hintergrund betrifft die vorliegende Erfindung auch ein Verfahren zur Herstellung einer spannungsfesten, elektrisch isolierenden Beschichtung auf einer Oberfläche eines chirurgischen Instruments, bei dem auf der Oberfläche nacheinander zumindest eine untere Schicht aus einem harten spannungsfesten Material in einer Stärke von höchstens 50 um und eine obere Schicht aus einem spaltgängigen spannungsfesten Material in einer Stärke von höchstens 50 um abgeschieden werden, wobei die Beschichtung eine Spannungsfestigkeit zumindest bis 1.000 Volt aufweist.

Die der Erfindung zugrundeliegende Aufgabe wird auf diese Weise vollkommen gelöst.

In bevorzugter Ausgestaltung weist die untere Schicht eine Lage aus DLC und/oder SiOₓ auf, ist bevorzugt zumindest aus zwei Lagen aufgebaut.

DLC und SiOₓ sind zwei für die untere Schicht besonders gut geeignete Materialien.

Dabei ist es bevorzugt wenn die untere Schicht eine Lage aus hartem DLC und eine Lage aus weichem DLC aufweist, wobei vorzugsweise die untere Schicht zumindest drei Lagen aus hartem DLC und drei Lagen aus weichem DLC aufweist, wobei die Lagen aus hartem und weichen DLC sich abwechseln, und weiter vorzugsweise die untere der Lagen aus hartem DLC besteht.

Hier ist von Vorteil, dass eine gut an der Oberfläche anhaftende mehrlagige DLC-Schicht geschaffen wird, die eine für eine einzelne Lage nicht ohne größere Defekte herstellbare Stärke aufweist, die so groß ist, dass eine Schicht aus dem Material der oberen Schicht in gleicher Stärke die erforderliche Spannungsfestigkeit aufweist.

Die Erfinder haben jedoch festgestellt, dass auch eine derart mehrlagige DLC-Schicht noch Defekte aufweist, die dazu führen, dass die Spanungsfestigkeit dieser Schicht die geforderten Werte nicht erreicht. Mit anderen Worten, die Defekte in den unterschiedlichen Lagen sind in der Regel nicht so verteilt, dass sie sich nicht überlappen. Erfindungsgemäß wird daher auch auf eine mehrlagige untere DLC-Schicht eine obere Schicht aus spaltgängigem Material aufgebracht.

Die Abscheidung von abwechselnd harten und weichen DLC-Lagen ist beispielsweise beschrieben in Knoblauch et al.,"Tribological properties of bias voltage modulated a-C:H nanoscaled multilayers", in Surface and Coatings Technology, 94-95 (1997), Seiten 521 - 524.

Die Autoren beschreiben, dass mit plasmaunterstützter chemischer Gasphasenabscheidung (PACVD) DLC-Schichten abgeschieden werden können, deren Härte von der Bias-Spannung abhängt.

Unter einer "Lage aus hartem DLC" wird eine DLC-Schicht verstanden, die mit einer Biasspannung abgeschieden wird, die mindestens doppelt so groß ist wie die Biasspannung, mit der die "Lage aus weichem DLC" abgeschieden wird. Die Angaben "hart" und "weich" sind also nicht als absolute Angaben sondern als relative Angaben zu verstehen.

In weiter bevorzugter Ausgestaltung weist jede Lage aus hartem oder weichem DLC eine Stärke auf, die zwischen 0,1 und 1,5, vorzugsweise zwischen 0,3 und 1 um liegt.

DLC-Lagen dieser Stärke lassen sich zuverlässig und mit guter Anhaftung auf üblichen Oberflächen und aufeinander abscheiden.

Dabei ist es bevorzugt, wenn die obere Schicht aus einem weichen Polymer, insbesondere aus Parylene besteht, und vorzugsweise eine Stärke aufweist, die zwischen 2 und 10 um liegt.

Parylene, insbesondere Parylene C und Parylene F sind zwei für die obere Schicht besonders gut geeignete Materialien, die Defekte in der unteren Schicht auch dann noch zuverlässig ausfüllen, wenn die untere Schicht eine Stärke von bis zu 20 um, vorzugsweise 10 um aufweist und mehrlagig ausgebildet ist.

Weiter ist es bevorzugt, wenn die obere Schicht eine Lage aus einem harten spannungsfesten Material, insbesondere aus Al₂O₃, SiOₓ, SiNₓ, Ta₂O₅, HfO₂, TiO₂ oder ZrO₂ aufweist, die mit einem ALD-Verfahren hergestellt ist, wobei die obere Schicht vorzugsweise eine Stärke aufweist, die geringer ist als 500 nm.

Trägt man harte Materialien mit den üblichen Verfahren zur Dünnschichterzeugung wie PVD (physikalische Gasabscheidung) oder PACVD (plasmaunterstützte chemische Gasphasenabscheidung) auf Substrate auf, so zeigen sie viele lokale Defekte wie Pinholes oder Spannungsrisse. Mit ALD, einem modifizierten CVD-Verfahren (chemische Gasphasenabscheidung), lassen sich die gleichen harten Materialien zwar Pinholefrei und Spannungsriss-frei auftragen, aber wirtschaftlich nicht in hinreichender Dicke, beispielsweise im Bereich von einigen µm.

Allerdings kann eine dünne ALD-Schicht genauso wie Parylene kleinere Defekte verschließen, da es sehr spaltgängig ist. Durch Kombination der Beschichtungsverfahren lassen sich größere Dicken wirtschaftlich herstellen.

Die Erfinder haben festgestellt, dass dünne ALD-Schichten von bis zu 500 nm in der Lage sind, die Defekte in der unteren Schicht so vollständig zu schließen, so dass die so aufgebaute Isolationsschicht die erforderliche Spannungsfestigkeit aufweist und mechanisch hinreichend beanspruchbar ist.

Die Vorrichtung ist ein chirurgisches, insbesondere ein elektrochirurgisches Instrument.

Die Erfinder haben mehrlagige dünne Isolationsschichten geschaffen, die eine hohe Spannungsfestigkeit von bis zu 1.000 V und sogar über 3.000 Volt aufweisen. Dabei wurden verschiedene Schichtsysteme aus DLC und SiOₓ mit einer oberen Schicht aus Parylene auf ihre Haftfestigkeit, Autoklavierbarkeit und Spannungsfestigkeit untersucht.

DLC (diamond-like carbon) ist ein diamantähnlicher Werkstoff, der sich von Diamant nur in der Art der Kohlenstoffbindung unterscheidet. Bei Diamant besitzen alle vier Valenzelektronen das gleiche Energieniveau, während bei DLC nur drei der vier Valenzelektronen dasselbe Energieniveau besitzen.

Man unterscheidet zwischen wasserstoffhaltigem und wasserstofffreiem DLC. Amorphe wasserstoffhaltige DLC-Schichten (a-C:H) lassen sich mittels einer Plasmapolymerisation herstellen. Ein mögliches Verfahren um derartige Schichten zu synthetisieren ist die plasmaunterstützte chemische Gasphasenabscheidung (PACVD); siehe US 5,512,330.

DLC-Schichten besitzen eine Kratz- und Verschleißfestigkeit, die fast so gut ist wie für Diamant, eine hohe Spannungsfestigkeit, eine sehr gute chemische Beständigkeit, eine herausragende Korrosionsbeständigkeit und eine gute Biokompatibilität. Andererseits weisen DLC-Schichten gute Gleiteigenschaften gegen Metall und gute Antihaftwirkung auf.

Es ist möglich DLC-Schichten zu erzeugen, die eine Dielektrizitätszahl zwischen 3,3 und 2,7 besitzen. Die Spannungsfestigkeit beträgt bei einer Schichtdicke von einem Mikrometer etwa 550 V/µm. Aufgrund dieser Eigenschaften wird DLC in vielen verschiedenen Bereichen eingesetzt.

In der Medizintechnik werden DLC-Beschichtungen für Implantate mit Blutkontakt wie zum Beispiel künstliche Herzklappen, Blutpumpen und Stents eingesetzt.

Außerdem wird die Korrosionsbeständigkeit und Antihaftwirkung genutzt, um Schutzschichten für minimalinvasive Chirurgieinstrumente herzustellen. In anderen Bereichen wird DLC als Schutzschicht für stark beanspruchte Maschinenelemente, Kugellager oder Zahnräder mit verminderter Reibung, Rasierklingen und als Verschleißschutz in der Automobilbranche verwendet.

DLC besitzt jedoch eine relativ hohe Eigenspannung, was die haftfest erzeugbare Schichtdicke und auch die Materialen begrenzt, auf denen DLC-Schichten abgeschieden werden können. Eigenspannungen sind Spannungen die in einem Material oder Bauteil auftreten, ohne Einwirkung von äußeren Kräften. Sie entstehen während der Herstellung durch plastische Verformung oder Temperaturänderungen und bleiben im Material zurück. Besonders bei Materialien mit deutlich unterschiedlichen Wärmeausdehnungskoeffizienten führt dies zu Haftungsproblemen und zur Ausbildung von Defekten, die die Spannungsfestigkeit beinträchtigen können.

Die US 2011/0122486 A1 beschreibt eine gegenüber Ionen in flüssigen Medien elektrisch isolierende und diffusionsdichte Beschichtung für beispielsweise Implantate. Die Beschichtung dient dazu, sowohl das Eindringen von Körperflüssigkeiten, körpereigenen Substanzen und Ionen in das Implantat als auch das Austreten von Bestandteilen aus dem Implantat zu verhindern.

Die Beschichtung umfasst eine mehrlagige obere Schicht, die bspw. aus abwechselnden, jeweils sehr dünnen Lagen aus diffusionsresistentem und nachgiebigem a-C:H besteht, und eine untere, substratnahe Schicht aus Parylene, die der Verbindung zu dem Substrat dient. Zwischen der oberen und der unteren Schicht sowie zwischen der unteren Schicht und dem Substrat ist jeweils eine Übergangsschicht angeordnet. Die äußere Lage der oberen Schicht besteht aus a-C:H mit 20% Wasserstoffgehalt. Die diffusionsresistenten Lagen sind dünner ausgebildet als die nachgiebigen Lagen von a-C:H.

Die Stärke der einzelnen Lagen der oberen Schicht und der Übergansschichten liegt zwischen 20 und 40 nm, in einem Ausführungsbeispiel sind 13 derartige Lagen vorgesehen, was zu einer Gesamtstärke der oberen Schicht von 345 nm führt. Die untere Schicht weist hier eine Stärke von 500 nm auf.

Die einzelnen Lagen der oberen Schicht werden so abgeschieden, dass sie keine Brüche oder andere Defekte aufweisen, die als Sacklöcher oder durchgehende Löcher (pinholes) der gewünschten Diffusionsdichtigkeit abträglich wären.

Die bekannte Beschichtung ist u.a. wegen der Forderung nach pinholefreien a-C:H Schichten aufwändig herzustellen und weist zudem nicht die erforderliche Spannungsfestigkeit auf, wie sie beispielsweise für chirurgische Instrumente benötigt wird.

Die US 6,926,572 B2 beschreibt ein flaches Display auf OLED-Basis, auf dessen Rückseite oder Vorderseite eine mehrlagige Passivierungsschicht aufgebracht ist, um das Display vor dem Eindringen von Sauerstoff und Feuchtigkeit zu schützen. Die Passivierungsschicht umfasst eine nicht genauer spezifizierte Abfolge aus abwechselnd abgeschiedenen organischen, anorganischen und metallischen Lagen Die organischen Lagen können Parylene umfassen und eine Stärke von 0,5 bis mehreren um aufweisen. Die anorganischen Lagen können SiO₂ oder durch plasmaverstärktes Atomic Layer Deposition (ALD) Verfahren abgeschiedenes Al₂O₃ mit einer Stärke von ca. 200 nm umfassen. Die metallischen Lagen können Al mit einer Stärke von 100 nm aufweisen.

Eine bevorzugte Abfolge oder Anordnung der einzelnen Lagen ist nicht angegeben.

Auch diese bekannte Beschichtung ist aufwändig herzustellen und weist zudem nicht die erforderliche Spannungsfestigkeit auf, wie sie beispielsweise für chirurgische Instrumente benötigt wird.

Die US 2001/0052752 A1 beschreibt eine Schutzschicht für ein Display auf OLED-Basis, wobei die Schutzschicht das Display vor dem Eindringen von Sauerstoff und Feuchtigkeit schützen soll. Die zweilagig aufgebaute Schutzschicht umfasst eine Oxidschicht, beispielsweise aus Al₂O₃ oder SiO₂, und eine Polymerschicht, beispielsweise aus Parylene, wobei entweder die Oxidschicht oder die Paryleneschicht als äußere, substratferne Schicht angeordnet ist. Die Oxidschicht soll so dünn sein, dass sie optisch transparent bleibt, beispielsweise eine Stärke von weniger als 100 nm aufweisen. Die Stärke der Paryleneschicht ist nicht explizit angegeben.

Auch diese bekannte Beschichtung weist nicht die erforderliche Spannungsfestigkeit auf, wie sie beispielsweise für chirurgische Instrumente benötigt wird. Zudem kann die sehr dünne Oxidschicht nicht die beispielsweise für chirurgische Instrumente erforderlichen mechanischen Eigenschaften erbringen.

DLC-Schichten wurden daher bisher nicht erfolgreich zur Beschichtung von elektrochirurgischen Instrumenten eingesetzt, weil keine hohe Spannungsfestigkeit zu erzielen war.

SiOₓ ist die Bezeichnung für eine chemische Verbindung zwischen Silizium und Sauerstoff. In der Natur tritt es sowohl in kristalliner als auch in amorpher Form auf. Siliziumdioxid ist ein wichtiges Material im Bereich der Halbleiter- und Mikrosystemtechnik. Dort wird es hauptsächlich als Isolator und Passivierungsschicht eingesetzt. Siliziumdioxid besitzt eine Dielektrizitätszahl εr von 3,9. Die Spannungsfestigkeit beträgt bei einer Schichtdicke von 50 nm ca. 1.000 V/µm.

Siliziumoxid-Schichten lassen sich ebenfalls mit dem PECVD-Verfahren herstellen. Ein für die Herstellung von SiOₓ verwendetes Gasgemisch besteht aus Hexamethyldisiloxan (HMDSO) und Sauerstoff.

Die Erfinder haben nun festgestellt, dass dünne Schichten aus DLC oder SiOₓ, die für sich nicht haftfest und defektfrei auf den Vorrichtungen abgeschieden werden können, dennoch für den Aufbau von erfindungsgemäßen Isolationsschichten verwendet werden können, wenn sie als untere Schicht eingesetzt und mit einer weiteren Schicht, vorzugsweise aus Parylene beschichtet werden.

Die Trägerstruktur für die Oberfläche der Vorrichtung, auf der die Beschichtung ausgebildet werden soll, ist vorzugsweise aus Metall, insbesondere Edelstahl, kann aber auch eine flexible Folie sein.

Die obere Parylene-Schicht dient dabei hauptsächlich dazu, eventuelle Defekte in den tiefer liegenden Isolationsschichten zu verschließen.

Parylene ist die Abkürzung für eine Gruppe von Poly-(para)-Xylenen, welche linear, teilkristallin und unvernetzt sind. Die Abscheidung von Parylene-Schichten findet aus der Gasphase statt und ermöglicht eine lufteinschlussfreie und gleichmäßige Abscheidung. Auf der gesamten Oberfläche findet eine Polymerisation durch Diffusionsreaktionen statt. Dadurch wird eine gleichmäßige Abscheidung erreicht. Selbst Ecken, Kanten und komplexe Geometrien werden bedeckt. Durch die exzellenten Diffusionseigenschaften des Monomers können auch kleine Löcher verschlossen werden. Es gibt vier industriell genutzte Parylenearten, die unterschiedliche Eigenschaften besitzen, nämlich Parylene C, Parylene D, Parylene N und Parylene F.

Parylene-Beschichtungen lassen sich homogen auch auf unregelmäßigen Oberflächengeometrien abscheiden, zeigen Barrierewirkung gegen z.B. Sauerstoff oder Wasser, wirken als Korrosionsschutz, sind elektrisch isolierend mit hoher Spannungsfestigkeit, lassen eine hohe mechanische Verformbarkeit zu und sind sterilisationsbeständig.

Es ist möglich Parylene-Schichten zu erzeugen, die eine Dielektrizitätszahl zwischen 3 und 2 besitzen. Die Spannungsfestigkeit beträgt bei einer Schichtdicke von einem Mikrometer etwa 220 V/µm.

Parylene wird daher bereits zur Beschichtung von biomedizinischen Geräten wie Herzschrittmachern, Stents und flexiblen neuronalen Sonden verwendet.

Parylene-Schichten sind wegen ihrer hohen mechanischen Verformbarkeit jedoch nicht hinreichend kratz- und verschleißfest, so dass sie trotz ihrer hohen Spannungsfestigkeit bisher nicht für die Beschichtung von elektrochirurgischen Instrumenten verwendet wurden.

Die Erfinder haben erkannt, dass die guten mechanischen und elektrischen Eigenschaften von DLC-Schichten, nämlich die hohe Kratz- und Verschleißfestigkeit sowie die hohe Spannungsfestigkeit, in einer mehrlagigen Beschichtung genutzt werden können, wenn die nachteiligen Eigenschaften, dass sie nicht ausreichenden haftfest und defektfrei auf Vorrichtungen abgeschieden werden können, durch eine Überschichtung mit Parylene ausgeglichen werden.

Umgekehrt lassen sich die guten elektrischen Eigenschaften von Parylene und dessen Sterilisierbarkeit in einer mehrlagigen Beschichtung nutzen, obwohl Parylene-Schichten nicht hinreichend abriebfest sind.

Als eine besonders gute Isolationsschicht stellte sich eine Kombination aus DLC-Multilagenschichten und Parylene F heraus. Dabei wurden in ersten Versuchen abwechselnd vergleichsweise harte und weiche DLC-Schichten mit einer Gesamtschichtdicke von 5 µm abgeschieden und anschließend mit einer 5 µm dicken Schicht Parylene F überdeckt.

Spannungsfestigkeitsmessungen bei Netzfrequenz ergaben, dass diese Isolationsschicht eine Spannungsfestigkeit von bis zu 1.000 V aufweist und somit die Anforderung der Norm 60601-2-2 erfüllt.

In weiteren Versuchen wurden Isolationsschichten mit abwechselnd vergleichsweise harten und weichen DLC-Schichten mit einer Gesamtschichtdicke von 11 µm bzw.15 µm abgeschieden und anschließend jeweils mit einer 8,6 µm dicken Schicht Parylene C überdeckt.

Spannungsfestigkeitsmessungen bei Netzfrequenz ergaben, dass die dickere der beiden Isolationsschichten eine Spannungsfestigkeit von über 3 000 V und die dünnere Isolationsschicht eine Spannungsfestigkeit von bis zu 2 600 Volt aufweist, so dass diese Isolationsschichten auch die Anforderung der Norm 60601-2-2 erfüllen.

Die Erfindung betrifft weiter ein chirurgisches, vorzugsweise elektrochirurgisches Instrument mit einer Oberfläche, auf der eine mehrlagig ausgebildete, zumindest bis 1.000 Volt spannungsfeste, elektrisch isolierende Beschichtung vorgesehen ist, die eine auf der Oberfläche angebrachte untere Schicht, die eine Stärke von höchstens 50 um aufweist, und eine auf der unteren Schicht angebrachte obere Schicht umfasst, die eine Stärke von höchstens 50 um aufweist, wobei die untere Schicht aus zumindest zwei Lagen eines harten spannungsfesten Materials und die obere Schicht aus einem spaltgängigen spannungsfesten Material besteht.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der beigefügten Zeichnung dargestellt und werden in der nachfolgenden Beschreibung näher erläutert. Es zeigen:
- Fig. 1: in schematischer, geschnittener Seitenansicht eine Vorrichtung mit spannungsfester, elektrisch isolierender Beschichtung; und
- Fig. 2: eine Darstellung wie in Fig. 1, jedoch mit einem anderen Aufbau der Beschichtung; und
- Fig. 3: ein Schema für eine Messanordnung, mit der die Spannungsfestigkeit eines mit der neuen Beschichtung versehenen medizinischen Instrumentes geprüft wurde.

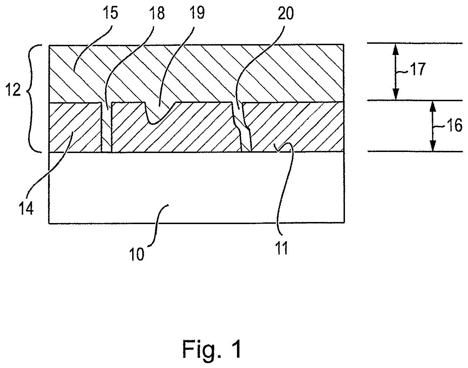

In Fig. 1 ist in schematischer und nicht maßstabsgemäßer Seitenansicht eine Vorrichtung 10 gezeigt, auf deren Oberfläche 11 eine zweilagige Beschichtung 12 aufgebracht ist. Die Vorrichtung 10 ist beispielhaft als Rechteck gezeigt. *In realiter* enthält sie eine Trägerstruktur, auf der sich die beschichtete Oberfläche 11 befindet. Als Material für die Trägerstruktur findet beispielsweise Edelstahl Anwendung.

Die Beschichtung 12 weist eine untere Schicht 14 und eine obere Schicht 15 auf. Die untere Schicht 14 weist eine bei 16 angedeutete Stärke und die obere Schicht 15 eine bei 17 angedeutet Stärke auf.

In der unteren Schicht 14 sind schematisch drei Defekte 18, 19 und 20 gezeigt, die durch Material der oberen Schicht 15 aufgefüllt wurden.

Die untere Schicht ist eine DLC-Schicht mit einer Stärke 16 von 1,5 um, die obere Schicht eine Schicht aus Parylene F mit einer Stärke von 10 um. Diese Beschichtung wies eine Spannungsfestigkeit von 550 Volt auf, was durch eine Erhöhung der Stärke 16 der DLC-Schicht auf über 1000 Volt gesteigert werden kann.

Für die Erzeugung der Parylene-Schicht 15 wurde in an sich bekannter Weise pulverförmiges di-para-Xylylen bei etwa 690° C in einem Paryleneverdampfer in den gasförmigen Zustand gebracht und dann in eine Vakuumkammer geleitet, wo es bei Raumtemperatur auf der DLC-Schicht kondensierte und die Parylene-Schicht bildete.

Die DLC-Schichten wurden in einer Niederdruck PECVD-Anlage in an sich bekannter Weise auf den Oberflächen abgeschieden. Die Anlage besteht aus einer Vakuumkammer, einem Pumpstand und einer regelbaren Gaszuführung, aus der ein Gasgemisch von Kohlenwasserstoffen (Tetramethylsilan und Acetylen) in die evakuierte Vakuumkammer geleitet wird, in der über zwei Elektroden, an die hochfrequente Biasspannung angelegt wird, ein Plasma erzeugt wird. Aus dem Plasma wird dann die DLC-Schicht auf der Oberfläche abgeschieden, wobei die Abscheiderate von dem Gasgemisch, dem Druck in der Vakuumkammer, und der Biasspannung abhängt.

Um die Stärke der DLC-Schicht 14 weiter zu steigern, wurden dann abwechselnde Lagen von harten DLC-Schichten und weichen DLC-Schichten abgeschieden, wobei die unmittelbar auf der Oberfläche 11 aufliegende Lage aus hartem DLC bestand. Es wurden insgesamt 4 harte und 4 weiche Lagen abgeschieden, was zu einer Stärke 16 von ca. 5 um führte.

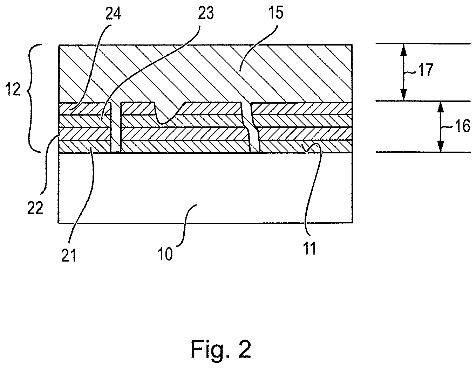

Diese Anordnung ist in Fig. 2 gezeigt, wobei dort beispielhaft vier Lagen 21, 22, 23, 24 in der Schicht 14 gezeigt sind, die Lagen 21 und 23 sind harte, die Lagen 22 und 24 weiche DLC-Lagen. Die Schicht 15 besteht aus Parylene F und weist eine Stärke 17 von 5 um auf.

Die harten DLC-Lagen wurden mit einer Biasspannung von 400 Volt, die weichen mit einer Biasspannung von 100 Volt in einer PAVCD-Anlage abgeschieden.

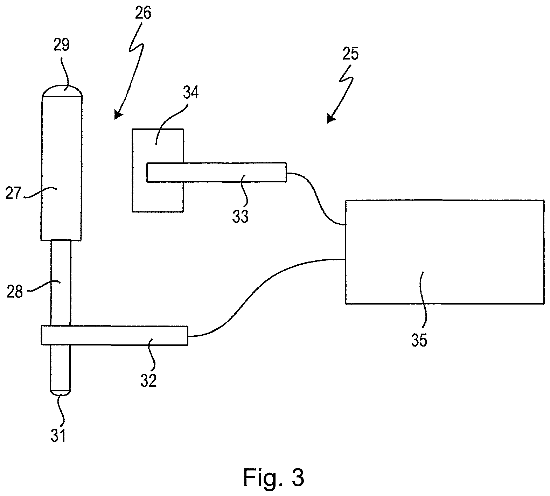

Die Spannungsfestigkeit der Beschichtung 12 aus Fig. 2 betrug in einem Messaufbau 25, wie er in Fig. 3 gezeigt ist, bis zu 1.100 Volt.

Als Messobjekt dient ein aus martensitischem Chromstahl mit der Kennung 1.4021 gefertigter Stab 26, der typische Geometrieformen für elektrochirurgische Instrumente repräsentiert. Er besteht aus einem Griff 27 mit einem Stiel 28, wobei oben an dem Griff 27 und unten an dem Stiel 28 markante Rundungen 29 und 31 ausgearbeitet sind.

Der Griff 27 hat eine Länge von 35 mm und einen Durchmesser von 10 mm, der Stiel eine Länge von 30 mm und einen Durchmesser von 4 mm.

Der Stab 26 wurde vollständig mit einer Beschichtung 12 versehen, wie sie anhand der Fig. 2 beschrieben wurde.

Eine erste Kontaktklemme 32 wird mit dem Stiel 28 und eine zweite kontaktklemme 33 über einen feuchten Schwamm 34 mit dem Griff 27 verbunden. Beide Kontaktklemme 32, 33 sind mit einem Prüfgerät 35 verbunden, das bei Netzfrequenz eine ansteigende Ausgangsspannungen bis 5.000 Volt liefert und den Spannungsdurchbruch anzeigt.

In diesen Versuchen wurden Spannungsfestigkeiten von 1.100 Volt festgestellt.

Die Beschichtung 12 war optisch ohne Beanstandung, haftete fest auf dem Stab 27 und zeigte bei ersten Manipulationen keinen Abrieb oder Verscheiß.

Die Haftung wurde mit dem Gitterschnittest nach DIN EN ISO 2409 bestimmt. Um eine beschleunigte Alterung zu bewirken, wurden die Messobjekte bei einer Temperatur von 60° C in PBS (Phosphat Buffered Saline) gelagert. Die Beschichtungen wurden zudem regelmäßig mit dem Klebebandabrisstest untersucht, bei dem Klebeband auf die Beschichtung aufgeklebt und unter einem Winkel von 60° abgezogen wird.

Für die hier beschriebenen Beschichtungen ergaben sich Haftfestigkeiten, die nach diesem Test in die beste Klasse GT0 (sehr gute Haftung) fallen.

Ferner wurden die Messobjekte autoklaviert, um ihre Sterilisierbarkeit zu überprüfen. Dazu wurden die Messobjekte für 3 Minuten einer Dampfsterilisierung bei 134° C unterzogen.

Die Versuche ergaben dass die Beschichtungen durch das Autoklavieren nicht beeinträchtigt wurden, insbesondere wurde die Haftfestigkeit dadurch nicht beeinträchtigt, was in nachgeschalteten Klebebandabrisstest verifiziert wurde.

## Patentansprüche

1. Chirurgisches Instrument mit einer Oberfläche (11), auf der eine elektrisch isolierende Beschichtung (12) vorgesehen ist, wobei die Beschichtung (12) mehrlagig ausgebildet ist, mit zumindest einer auf der Oberfläche aufgebrachten unteren Schicht (14), die eine Stärke (16) von höchstens 50 µm aufweist, und einer auf die untere Schicht aufgebrachten oberen Schicht (15), die eine Stärke (17) von höchstens 50 µm aufweist, **dadurch gekennzeichnet, dass** die untere Schicht (14) aus einem harten spannungsfesten Material und die obere Schicht (15) aus einem spaltgängigen spannungsfesten Material besteht, wobei die elektrisch isolierende Beschichtung (12) eine Spannungsfestigkeit zumindest bis 1.000 Volt aufweist.

2. Chirurgisches Instrument nach Anspruch 1, **dadurch gekennzeichnet, dass** die untere Schicht (14) eine Lage aus DLC (diamond-like carbon) aufweist.

3. Chirurgisches Instrument nach Anspruch 1, **dadurch gekennzeichnet, dass** die untere Schicht (14) eine Lage aus Al₂O₃, SiOₓ, SiNₓ, Ta₂O₅, HfO₂, TiO₂ oder ZrO₂ aufweist.

4. Chirurgisches Instrument nach einem der Ansprüche 1 bis 3, **dadurch gekennzeichnet**, die untere Schicht (14) aus zumindest zwei Lagen aufgebaut ist.

5. Chirurgisches Instrument nach Anspruch 4, **dadurch gekennzeichnet, dass** die untere Schicht (14) eine Lage (21, 23) aus hartem DLC und eine Lage (22, 24) aus weichem DLC aufweist.

6. Chirurgisches Instrument nach Anspruch 5, **dadurch gekennzeichnet, dass** die untere Schicht (14) zumindest drei Lagen (21, 23) aus hartem DLC und drei Lagen (22, 24) aus weichem DLC aufweist, wobei die Lagen aus hartem und weichen DLC sich abwechseln.

7. Chirurgisches Instrument nach Anspruch 6, **dadurch gekennzeichnet, dass** die untere der Lagen aus hartem DLC besteht.

8. Chirurgisches Instrument nach einem der Ansprüche 2 bis 7, **dadurch gekennzeichnet, dass** jede Lage (21, 22, 23, 24) aus DLC eine Stärke aufweist, die zwischen 0,1 und 1,5 µm, vorzugsweise zwischen 0,3 und 1 µm liegt.

9. Chirurgisches Instrument nach einem der Ansprüche 1 bis 8, **dadurch gekennzeichnet, dass** die obere Schicht (15) aus Parylene besteht.

10. Chirurgisches Instrument nach Anspruch 9, **dadurch gekennzeichnet, dass** die obere Schicht (15) eine Stärke (17) aufweist, die zwischen 2 und 10 µm

11. Chirurgisches Instrument nach einem der Ansprüche 1 bis 8, **dadurch gekennzeichnet, dass** die obere Schicht (15) eine Lage aus Al₂O₃, SiOₓ, SiNₓ, Ta₂O₅, HfO₂, TiO₂ oder ZrO₂ aufweist, die mit einem Atomic Layer Deposition-Verfahren (ALD-Verfahren) hergestellt ist.

12. Chirurgisches Instrument nach Anspruch 11, **dadurch gekennzeichnet, dass** die obere Schicht (15) eine Stärke (17) aufweist, die geringer ist als 500 nm.

13. Verfahren zur Herstellung einer elektrisch isolierenden Beschichtung (12) auf einer Oberfläche (11) eines chirurgischen Instruments, bei dem auf der Oberfläche (11) nacheinander zumindest eine untere Schicht (14) aus einem harten spannungsfesten Material in einer Stärke (16) von höchstens 50 µm und eine obere Schicht (15) aus einem spaltgängigen spannungsfesten Material in einer Stärke (17) von höchstens 50 µm abgeschieden werden, wobei die elektrisch isolierende Beschichtung (12) eine Spannungsfestigkeit zumindest bis 1.000 Volt aufweist.

## Claims

1. Surgical instrument comprising a surface (11) provided with an electrically insulating coating (12),
wherein the coating (12) is a multilayer coating, comprising at least one bottom layer (14) having a thickness (16) of at most 50 µm applied on the surface, and comprising a top layer (15) having a thickness (17) of at most 50 µm applied on the bottom layer,
**characterized in that** the bottom layer (14) consists of a hard voltage-resistant material and the top layer (15) consists of a gap-penetrative voltage-resistant material, wherein the electrically insulating coating (12) is voltage-resistant at least up to 1000 volts.

2. Surgical instrument as claimed in claim 1, **characterized in that** the bottom layer (14) comprises a layer of DLC (diamond-like carbon).

3. Surgical instrument as claimed in claim 1, **characterized in that** the bottom layer (14) comprises a layer of Al₂O₃, SiOₓ, SiNₓ, Ta₂O₅, HfO₂, TiO₂, or ZrO₂.

4. Surgical instrument as claimed in any of claims 1 to 3, **characterized in** the bottom layer (14) is constructed of at least two layers.

5. Surgical instrument as claimed in claim 4, **characterized in that** the bottom layer (14) comprises a layer (21, 23) of hard DLC and a layer (22, 24) of soft DLC.

6. Surgical instrument as claimed in claim 5, **characterized in that** the bottom layer (14) comprises at least three layers (21, 23) of hard DLC and three layers (22, 24) of soft DLC, the layers of hard and soft DLC being in alternation.

7. Surgical instrument as claimed in claim 6, **characterized in that** the lower of the layers consists of hard DLC.

8. Surgical instrument as claimed in any of claims 2 to 7, **characterized in that** each layer (21, 22, 23, 24) of DLC has a thickness of between 0.1 and 1.5 µm, preferably between 0.3 and 1 µm.

9. Surgical instrument as claimed in any of claims 1 to 8, **characterized in that** the top layer (15) consists of parylene.

10. Surgical instrument as claimed in claim 9, **characterized in that** the top layer (15) has a thickness (17) of between 2 and 10 µm.

11. Surgical instrument as claimed in any of claims 1 to 8, **characterized in that** the top layer (15) comprises a layer of Al₂O₃, SiOₓ, SiNₓ, Ta₂O₅, HfO₂, TiO₂, or ZrO₂, which is produced by an atomic layer deposition process (ALD process).

12. Surgical instrument as claimed in claim 11, **characterized in that** the top layer (15) has a thickness (17) of less than 500 nm.

13. Method for producing an electrically insulating coating (12) on a surface (11) of a surgical instrument (10), wherein there are deposited in succession on the surface (11) at least one bottom layer (14) of a hard voltage-resistant material in a thickness (16) of at most 50 µm and a top layer (15) of a gap-penetrative voltage-resistant material in a thickness (17) of at most 50 µm, wherein the electrically insulating coating (12) is voltage-resistant at least up to 1000 volts.

## Revendications

1. Instrument chirurgical comprenant une surface (11), sur laquelle un revêtement électriquement isolant (12) est prévu, le revêtement (12) étant configuré à plusieurs couches avec au moins une couche inférieure (14) appliquée sur la surface, qui présente une épaisseur (16) d'au plus 50 µm, et une couche supérieure (15) appliquée sur la couche inférieure, qui présente une épaisseur (17) d'au plus 50 µm, **caractérisé en ce que** la couche inférieure (14) est constituée par un matériau dur résistant aux tensions et la couche supérieure (15) est constituée par un matériau pénétrant dans les interstices et résistant aux tensions, le revêtement électriquement isolant (12) présentant une résistance à des tensions d'au moins jusqu'à 1 000 volt.

2. Instrument chirurgical selon la revendication 1, **caractérisé en ce que** la couche inférieure (14) comprend une couche en DLC (diamond-like carbon).

3. Instrument chirurgical selon la revendication 1, **caractérisé en ce que** la couche inférieure (14) comprend une couche en Al₂O₃, SiOₓ, SiNₓ, Ta₂O₅, HfO₂, TiO₂ ou ZrO₂.

4. Instrument chirurgical selon l'une quelconque des revendications 1 à 3, **caractérisé en ce que** la couche inférieure (14) est formée par au moins deux couches.

5. Instrument chirurgical selon la revendication 4, **caractérisé en ce que** la couche inférieure (14) comprend une couche (21, 23) en DLC dur et une couche (22, 24) en DLC mou.

6. Instrument chirurgical selon la revendication 5, **caractérisé en ce que** la couche inférieure (14) comprend au moins trois couches (21, 23) en DLC dur et trois couches (22, 24) en DLC mou, les couches en DLC dur et mou étant alternées.

7. Instrument chirurgical selon la revendication 6, **caractérisé en ce que** la couche inférieure est constituée par du DLC dur.

8. Instrument chirurgical selon l'une quelconque des revendications 2 à 7, **caractérisé en ce que** chaque couche (21, 22, 23, 24) en DLC présente une épaisseur qui est comprise entre 0,1 et 1,5 µm, de préférence entre 0,3 et 1 µm.

9. Instrument chirurgical selon l'une quelconque des revendications 1 à 8, **caractérisé en ce que** la couche supérieure (15) est constituée par du parylène.

10. Instrument chirurgical selon la revendication 9, **caractérisé en ce que** la couche supérieure (15) présente une épaisseur (17) qui est comprise entre 2 et 10 µm.

11. Instrument chirurgical selon l'une quelconque des revendications 1 à 8, **caractérisé en ce que** la couche supérieure (15) comprend une couche en Al₂O₃, SiOₓ, SiNₓ, Ta₂O₅, HfO₂, TiO₂ ou ZrO₂ qui est fabriquée par un procédé ALD (Atomic Layer Déposition).

12. Instrument chirurgical selon la revendication 11, **caractérisé en ce que** la couche supérieure (15) présente une épaisseur (17) qui est inférieure à 500 nm.

13. Procédé de fabrication d'un revêtement électriquement isolant (12) sur une surface (11) d'un instrument chirurgical, selon lequel au moins une couche inférieure (14) en un matériau dur résistant aux tensions en une épaisseur (16) d'au plus 50 µm et une couche supérieure (15) en un matériau pénétrant dans les interstices et résistant aux tensions en une épaisseur (17) d'au plus 50 µm sont déposées successivement sur la surface (11), le revêtement électriquement isolant (12) présentant une résistance à des tensions d'au moins jusqu'à 1 000 volt.
